# EUROPEAN PATENT SPECIFICATION

(11) **EP 3 895 949 B1**
(45) Date of publication and mention of the grant of the patent: **16.08.2023**
(21) Application number: 20315192.3
(22) Date of filing: 17.04.2020
(51) Int. Cl.: B60W 40/08

(54) **METHOD AND DEVICE FOR EVALUATING USER DISCOMFORT**
VERFAHREN UND VORRICHTUNG ZUR BEWERTUNG DES BENUTZERKOMFORTS
PROCÉDÉ ET DISPOSITIF D'ÉVALUATION DE LA GÊNE D'UN UTILISATEUR

(43) Date of publication of application: 20.10.2021
(73) Proprietor: TOYOTA JIDOSHA KABUSHIKI KAISHA, TOYOTA-SHI, AICHI-KEN 471-8571 (JP); Université Gustave Eiffel, 77454 Marne-la-Vallée Cedex 2 (FR)
(72) Inventor: De Weser, Marleen, 1140 Brussels (BE); Jallais, Christophe, 69500 Bron (FR); Berthelon, Catherine, 13300 Salon-de-Provence (FR); Fort, Alexandra, 69500 Bron (FR); Hidalgo, Antonio, 31400 Toulouse (FR); Bequet, Adolphe, 69500 Bron (FR); Moreau, Fabien, 69500 Bron (FR); Tattegrain, Hélène, 69500 Bron (FR); Evin, Morgane, 13015 Marseille Cedex (FR)
(74) Representative: Cabinet Beau de Loménie

(56) References cited:
- EP-A1- 3 620 971
- WO-A1-2018/211301
- DE-A1-102016 215 250
- US-A1- 2016 236 690
- MUBASHER MIAN MUHAMMAD ET AL: "Modeling of individual differences in car-following behaviour of drivers", 2017 INTERNATIONAL MULTI-TOPIC CONFERENCE (INMIC), IEEE, 24 November 2017 (2017-11-24), pages 1-7, XP033318957, DOI: 10.1109/INMIC.2017.8289478 [retrieved on 2018-02-09]
- FAN XINMIAO ET AL: "A personalized traffic simulation integrating emotion using a driving simulator", VISUAL COMPUTER, SPRINGER, BERLIN, DE, vol. 36, no. 6, 24 July 2019 (2019-07-24), pages 1203-1218, XP037109573, ISSN: 0178-2789, DOI: 10.1007/S00371-019-01732-4 [retrieved on 2019-07-24]

## Description

### TECHNICAL FIELD

The invention relates to a computer-implemented method for evaluating user discomfort and to a data-processing device configured to carry out this method.

### BACKGROUND

The automotive industry is increasingly introducing Advanced Driver Assistance Systems (ADAS) designed to improve driving safety by reducing the risk of human error. These embedded systems can interact with the driver in different ways, either by giving the driver additional information on the state of the environment (via a multi-modal communication interface: audio, visual, tactile, etc.), or by processing information about the driver's mental state such as stress, fatigue, vigilance or drowsiness in order to assist him and/or to prevent potential risks (cf. Jinjun Wang et al, 2010: "Real-time driving danger-level prediction", Engineering Applications of Artificial Intelligence; Volume 23, Issue 8, December 2010, Pages 1247-1254). These existing systems process individually stress, fatigue, vigilance or drowsiness by considering behavioural or physiological parameters separately.

However, it is desirable to not only measure one specific mental state of the driver (fatigue or drowsiness, etc.) but to measure the feeling or experience of discomfort in driving. Meng and Siren (2012) propose that discomfort in driving can be considered as a form of awareness of the driver of changes in his/her own driving ability (cf. Meng and Siren (2012): "Cognitive problems, self-rated changes in driving skills, driving-related discomfort and self-regulation of driving in old drivers" Accident Analysis & Prevention, Volume 49, November 2012, Pages 322-329). Thus, these authors define the feeling of discomfort as related to a feeling of anxiety as a consequence of being self-aware that one's driving ability in a specific driving situation (for example driving in heavy rain) may include a potential driving risk. This feeling of discomfort may increase in time and can lead to complete avoidance of specific driving situations. However, Meng and Siren only analyze subjective data using questionnaires without considering measuring the (objective) driver behavioral or physiological data. In addition, most of the research dealing with (dis)comfort in driving, addresses this notion mainly in terms of physical ergonomics of the driving cabin (posture, driver seat dimensions, etc.).

It is further known to measure the driver's mental state by electrophysiological and/or driver behavioral measures. For example, WO2008/0127465 A1 discloses a system which predicts driving danger by capturing vehicle dynamic parameters, driver physiological data and driver behavior features; applying a learning algorithm to the features; and predicting driving danger. However, the system only predicts the driving danger of the current driving situation, in order to warn a driver before an undesirable event as e.g. an accident happens. It has also been proposed, for example in KR 20170094836 A, US 2018/0174457, CN 109017797 or CN 107662611 A, to measure the emotional state of the driver, on the basis of physiological data, driving behavior and/or emotional cues, and eventually to change the driving mode accordingly. On the other hand, in WO 2018/014953 A1, it was proposed to use physiological and behavioral data, together with vehicle context data, to determine a discomfort level index value, and output a warning and/or perform driver assistance if this value exceeds a reference index. Finally, US 2016/236690 A discloses a voice interaction system providing different output to a driver depending on driver state, vehicle state and environment, wherein the driver state may include physiological, cognitive and affective aspects, e.g. alertness and emotional state.

However, these previously proposed devices and methods fail to take into consideration endogenous psychological factors which are not necessarily reflected in physiological or behavioral data or even in emotional cues, but will influence the driver's subjective discomfort, with a potentially serious impact on driving safety.

### SUMMARY

A first aspect of the present invention relates to a data processing device configured to be connected to one or more physiological sensors for receiving physiological data of a user, to one or more behavioral sensors for receiving behavioral data of the user and to a vehicle context detection unit for receiving vehicle context information, and to determine a discomfort level index value based on a personality profile of the user together with at least the vehicle context information and physiological and behavioral data received within corresponding time windows. The discomfort level index value may be an integer from a scale graded by either ascending or descending level of discomfort. It could thus be alternatively understood as a comfort index level value. The user may be a vehicle driver or passenger, for example in a partially or fully automated vehicle.

Consequently, the endogenous influence of the user's own personality on his subjective discomfort level can be more adequately reflected, together with exogenous factors such as the vehicle context, on the discomfort level index value determined by the data processing device.

The data processing device may be configured to be also connected to an emotional state detection unit for receiving emotional state information concerning the user, and to determine the discomfort level index value based also on the emotional state information concerning the user. In particular, the emotional state detection unit may be configured to detect emotional cues in images and/or sound received from a camera and/or a microphone connected thereto. Consequently, not only the user's baseline personality profile may be reflected in the discomfort level index value, but also his transient emotional state.

The personality profile may comprise an index value associated to each of one or more personality traits, which may in particular include openness, agreeableness, extraversion, neuroticism, and/or conscientiousness, that is, the "Big Five" personality traits, and/or trait anxiety as measured for instance by the State-Trait Anxiety Inventory (STAI).

The data processing device may be configured to apply a learning classification algorithm, such as e.g. any one of random forest, J48, naive Bayesian, deep neural network or recurrent neural network algorithms, for determining the discomfort level index value. Consequently, this algorithm may be first taught in a learning phase to establish correlations between discomfort level and vehicle context information, physiological and behavioral data and personality profile, as well as eventually also emotional state data, in a learning phase using experimental data from one or more user subjects and one or more driving events, so as to subsequently apply those correlations in determining the comfort level index value on the basis of those parameters.

The one or more physiological sensors may include one or more electro-dermal sensors, and the data processing device may be configured to receive, as physiological data from the one or more electro-dermal sensors, amplitude and/or latency of skin conductivity responses. The data processing device may in particular be configured to determine the discomfort index value on the basis of i.a. the number of occurrences of phasic skin conductivity responses within a corresponding time window, their mean amplitude, the standard deviation of their amplitude, their mean latency and/or the standard deviation of their latency.

Alternatively or complementarily to the electro-dermal sensors, the one or more physiological sensors may include one or more cardiac sensors, and the data processing device may be configured to receive RR intervals as physiological data from the one or more cardiac sensors. The data processing device may in particular be configured to determine the discomfort index value on the basis of i.a. the minimum RR interval, the maximum RR interval, the mean RR interval, the standard deviation of the RR intervals, and/or the square-rooted standard deviation of the RR intervals, within a corresponding time window.

Alternatively or complementarily to the electro-dermal sensors and/or cardiac sensors, the one or more physiological sensors may include one or more eye tracking sensors, and the data processing device may be configured to receive occurrence and/or duration of eye fixations as physiological data from the one or more eye tracking sensors. The data processing device may in particular be configured to determine the discomfort index value on the basis of i.a. the number of occurrences of eye fixations within a corresponding time window, their total duration, their mean duration, and/or the standard deviation of their duration.

The data processing device may in particular be also configured to output a warning and/or perform user assistance if the discomfort level index value exceeds a predetermined threshold. This may help ensure the user's comfort following future driving events, by preventing a negative long-term impact on the emotional and cognitive state of the user.

A second aspect of the present invention relates to a human-machine interface system comprising the data processing device of the first aspect, as well as the one or more physiological sensors, the one or more behavioral sensors, and the vehicle context detection unit, each connected to the data processing device.

A third aspect of the present invention relates to a vehicle comprising the human-machine interface system of the second aspect.

A fourth aspect of the present invention relates to a computer-implemented method for evaluating user discomfort, comprising the steps of receiving physiological data of a user from one or more physiological sensors; receiving behavioral data of the user from one or more behavioral sensors; receiving vehicle context information from a vehicle context detection unit; and determining a discomfort level index value based on a personality profile of the user, together with at least the physiological and behavioral data and vehicle context information received within corresponding time windows.

A fifth aspect of the present invention relates to a computer program comprising instructions which, when the program is executed by a computer, cause the computer to perform the computer-implemented method of the fourth aspect.

A sixth aspect of the present invention relates to computer-readable medium comprising instructions which, when executed by a computer, cause the computer to carry out the computer-implemented method of the fourth aspect.

### BRIEF DESCRIPTION OF THE DRAWINGS

The invention may be more completely understood in consideration of the following detailed description of various embodiments in connection with the accompanying drawing, which shows a block diagram of a vehicle according to embodiments of the present invention.

While the invention is amenable to various modifications and alternative forms, specifics thereof have been shown by way of example in the drawings and will be described in detail. It should be understood, however, that the intention is not to limit aspects of the invention to the particular embodiments described.

### DETAILED DESCRIPTION

For the following defined terms, these definitions shall be applied, unless a different definition is given in the claims or elsewhere in this specification.

Although some suitable dimension ranges and/or values pertaining to various components, features and/or specifications are disclosed, one of skill in the art, incited by the present disclosure, would understand desired dimensions, ranges and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The detailed description and the drawings, which are not necessarily to scale, depict illustrative embodiments and are not intended to limit the scope of the invention. The illustrative embodiments depicted are intended only as exemplary. Selected features of any illustrative embodiment may be incorporated into an additional embodiment unless clearly stated to the contrary.

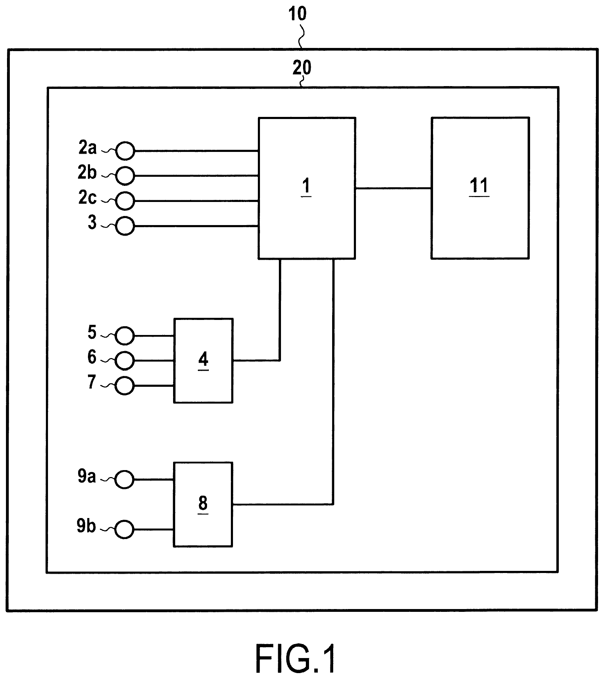

Fig. 1 shows a block diagram of a data processing device 1 according to embodiments of the present invention. The data processing device 1 may be part of a human-machine interface (HMI) system 20. The HMI system 20 may be part of a vehicle 10, and in particular provide a driving interface for a human user of the vehicle 10.

The data processing device 1 may be connected to and/or comprise a data storage for storing a reference data set. The data processing device 1 may comprise an electronic circuit, a processor (shared, dedicated, or group), a combinational logic circuit, a memory that executes one or more software programs, and/or other suitable components that provide the described functionality. The data processing device 1 may be configured to additionally carry out further functions in the vehicle 10. For example, the data processing device 1 may also be configured to act as a general purpose electronic control unit (ECU) of the vehicle 10.

The HMI system 20 may also comprise one or several physiological sensors 2a,2b,2c to which the data processing device 1 may be connected to receive a data output thereof. Each physiological sensor 2 may be configured to sense at least one physiological feature of the user. For example, the physiological sensors 2a,2b,2c electro-dermal may include one or more electro-dermal sensors 2a, one or more cardiac sensors 2b, and/or one or more eye tracking sensors 2c. The one or more electro-dermal sensors 2a may comprise Ag/AgCl electrodes, located for example at least at the level of the second phalanx of the index and the third digit of the non-dominant hand of the user and be configured to measure a skin conductance level (SCL). The one or more cardiac sensors 2b may also comprise Ag/AgCl electrodes and be configured to measure an electrocardiographic (ECG) signal. The one or more eye tracking sensors 2c may comprise, for instance, eye tracking Pupilabs^{®} v2 glasses, with a tracking frequency of 120 Hz, a gaze accuracy of 1.0°, a gaze precision of 0.08°, a camera latency of 5.7 ms, and a processing latency of 3-4 ms.

The SCL may be sampled, for instance, at a 1000 Hz sampling rate, and the one or more electro-dermal sensors 2a may be further configured to detect, from the SCL signal, the occurrence of phasic skin conductivity responses and measure their amplitude and/or their latency to transmit these, as physiological data, to the data processing device 1. Alternatively, however, the one or more electro-dermal sensors 2a may directly transmit the SCL signal to the data processing device 1, which may then be itself configured to detect the occurrence of phasic skin conductivity responses and measure their amplitude and/or their latency.

The ECG signal may also be sampled, for instance, at a 1000 Hz sampling rate, and the one or more cardiac sensors 2b may be further configured to detect, from the ECG signal, the individual heartbeats and measure the RR intervals between them, to transmit these, as physiological data, to the data processing device. Alternatively, however, the one or more cardiac sensors 2b may directly transmit the ECG signal to the data processing device 1, which may then be itself configured to detect the individual heartbeats and measure the RR intervals between them.

The eye tracking sensors 2c may be configured to detect occurrence and duration of eye fixations, and transmit them, as physiological data, to the data processing device 1. Alternatively however, the eye tracking sensors 2c may directly transmit pupil position data to the data processing device, which may be configured to detect occurrence and duration of eye fixations from these eye position data.

The HMI system 20 may also comprise one or more behavioral sensors 3. The one or more behavioral sensors 3 may be configured to measure one or more behavioral features of the user, in particular concerning driving behaviour, and transmit it to the data processing device 1. For example, the one or more behavioral sensors 3 may measure the vehicle's lateral position on the road (e.g. supported by GPS) and/or steering wheel angle. All behavioural data may be sampled at a 60Hz sampling rate.

Moreover, the HMI system 20 may also comprise a vehicle context detection unit 4. The vehicle context detection unit 4 may be configured to determine vehicle context information describing a current driving situation and transmit it to the data processing device 1. Such driving situations may include e.g. a situation in which the vehicle 10 comprising the HMI system 20 overtakes another vehicle, a situation in the vehicle 10 comprising the HMI system 20 is approaching a traffic light, a situation in which the vehicle 10 comprising the HMI system 20 approaches a pedestrian or a pedestrian approaches the vehicle 10, a situation in the vehicle 10 comprising the HMI system 20 is driven in the dark, a situation in the vehicle 10 comprising the HMI system 20 makes a left-turn (in right-hand traffic), a situation in which the vehicle 10 comprising the HMI system 20 is in dense traffic and/or facing aggressive and/or unexpected behavior by other road users or other driving situations which may challenge the comfort level of the user, such as, for instance heavy rain, snowfall or icing.

In order to determine the vehicle context information, the vehicle context detection unit 4 may be connected to a plurality of sensors configured to monitor the environment of the vehicle, e.g. a GPS 5, a radar 6 or one or several outside cameras 7. The vehicle context detection unit 4 may also be connected to the one or more behavioral sensors 3 for receiving their output. For example, based on the information received from a GPS, cameras and an electronic map providing a scheduled driving route, the vehicle context detection unit 4 can determine a left turn of the vehicle.

The HMI system 20 may also comprise an emotional state detection unit 8. The emotional state detection unit 8 may be configured to determine emotional state information describing a current emotional state of the user and transmit it to the data processing device 1. Such emotional state information may comprise an emotional state index value associated to each one of one or more emotions, such as, e.g. sadness, interest, anxiety, alertness, boredom, impatience, anger, serenity, fear and/or happiness.

In order to determine the emotional state information, the emotional state detection unit 8 may be connected to one or more sensors configured to monitor the emotional state of the user, e.g. an interior camera 9a and/or a microphone 9b, in order to receive information from them, such as images and/or sound, containing emotional cues. Such emotional cues may be contained e.g. in acoustic features of speech like timbre, prosody and rhythm and/or visual features linked to facial expression. These acoustic and/or visual features may be correlated to specific emotions using machine learning algorithms trained on datasets such as e.g. the Danish Emotional Speech database, the Berlin Database of Emotional Speech, and/or the AffectNet annotated facial expression database, so that the emotional state detection unit 8 may infer the emotional state information from the images and/or sound received from the camera 9a and/or microphone 9b. Alternatively or complementarily to those sensors, the emotional state detection unit 8 may also be connected to the physiological and/or behavioral sensors for receiving their output and using it to infer the emotional state information.

The vehicle context detection unit 4 and/or the emotional state detection unit 8 may each be an electronic control device, as e.g. a processor. Each one of them, or both, may also be provided by the general purpose ECU of the vehicle. It is further possible that the data processing device 1 comprises the vehicle context detection unit 4 and/or the emotional state detection unit 8.

The data processing device 1 may be configured to calculate, based on physiological data received from the one or more electro-dermal sensors 2a over an electro-dermal time window TW_EDA, i.a. the number N_scrs, mean amplitude Amp_moy, standard deviation of the amplitude Et_Amp_Scrs, mean latency Lat_Moy_scrs and/or standard deviation of the latency ET_lat_scrs of the phasic skin conductivity responses within this time window TW_EDA. Furthermore, the data processing device 1 may be configured to calculate, based on physiological data received from the one or more cardiac sensors 2b over a cardiac time window TW_ECG, i.a. the minimum RR interval RRint_min, the maximum RR interval RRint_max, the mean RR interval RRint_moy, the standard deviation SDNN of the RR intervals, and/or the room-mean-squared standard deviation RMSSD of the RR intervals, within this cardiac time window TW_ECG. Additionally or alternatively, the data processing device 1 may be configured to calculate, based on physiological data received from the one or more eye tracking sensors 2c over an eye tracking time window TW_ET, i.a. the number nbFix of occurrences of eye fixations within this eye tracking time window TW_ET, their total duration durationFixTot, their mean duration durationFixMean, and/or the standard deviation durationFixSD of their duration.

Furthermore, the data processing device 1 may be configured to calculate, based on behavioral data received from the one or more behavioural sensors 3 within a behavioural time window TW_BE, i.a. the standard deviation SDLP of the vehicle's lateral position on the road, the standard deviation SDWA of the steering wheel angle, and/or the steering wheel reversal rate SRR.

A personality profile of the user may be stored within the data storage connected to and/or comprised within the data processing device 1. This personality profile may comprise an index value associated to each of one or more personality traits, such as openness, agreeableness, extraversion, neuroticism, conscientiousness, and/or trait anxiety and may have been previously calculated e.g. on the basis of responses of the user to a questionnaire and/or physiological, behavioral, emotional and/or vehicle context data gathered over one or more drives and correlated to one or more of the personality traits by a learning algorithm. This learning algorithm may have been trained and optimized to select significantly correlated features from which the personality traits can be inferred on the basis of a generic dataset with data from multiple users in multiple situations.

The data processing device 1 may be configured to determine a vehicle context event based on the vehicle context information, and then a discomfort index level value for the discomfort resulting from this vehicle context event. Such vehicle context events may be e.g. overtaking another vehicle, approaching a traffic light, approaching a pedestrian, driving in the dark, or making a left-turn (in right- hand traffic). The discomfort level index value may take the form of an integer, e.g. an integer value between 1 and 5, between 1 and 3, or just one of 1 or 2.

More specifically, the data processing device 1 may be configured to apply a learning classification algorithm, such as e.g. a random forest, J48,naive Bayesian, deep neural network or recurrent neural network algorithm, in order to determine the discomfort level index value based on the personality profile of the user together with the vehicle context information received from the vehicle context detection unit 4, the physiological and behavioral data received, within the corresponding time windows TW_EDA, TW_ECG, TW_ET and TW_BE, from the physiological and behavioral sensors 2a-2c and 3, as well as, eventually, the emotional state information received from the emotional state detection unit 8. The learning classification algorithm may have been trained and/or updated using a generic dataset linked to multiple users and/or one specifically linked to the current user. This learning classification algorithm may have been chosen and optimized based on an index created from a weighting of available indices such as the AUC-ROC curve, precision, recall, TF/FP rate, Matthews correlation coefficient, and/or precision recall curves and subsequently optimized selecting the input features and corresponding time windows best correlated to the discomfort level index value.

The data processing device 1 may be further configured to output a warning and/or trigger user assistance if the discomfort level index value exceeds a predetermined threshold DT. For this purpose, the data processing device may for instance be connected to an advanced user assistance system (ADAS) 11 within the HMI system 20. The ADAS 11 may even be configured to gradually increase driving assistance, i.e. increasingly take over driving tasks, as the discomfort level index value determined by the data processing device increases, i.e. the user's discomfort increases. The ADAS 11 may even be capable of automated driving, e.g. level 2 partial automation, level 3 conditional automation, or even higher.

Three different methods for evaluating user discomfort have been tested in a driving simulator using correspondingly configured HMI systems 20. In each example, a different learning classification algorithm and different sets of physiological and behavioral data and personality traits have been applied. In each case, the learning classification algorithm has been trained on 59 different user test subjects. The three methods were then tested on 10 different user test subjects for validation. During both training and validation, the test subjects were exposed to various driving situations and asked to indicate their resulting discomfort level on a scale of 1 to 5. Their personality profiles were established using questionnaires.

In the first example, the data processing device 1 determined the discomfort level index value using a random forest algorithm, based on the parameters and corresponding time windows presented in Table 1:

| Electro-dermal | Cardiac | Eye-tracking | Behavioral | Personality |
|---|---|---|---|---|
| 0-60 s | 0-60 s | 0-30 s | 0-60 s | |
| N_scrs | SDNN | nbFix | SDLP | Openness |
| Amp_moy | | durationFixTot | SDWA | |
| Et_Amp_scrs | | durationFixMean | | |
| Lat_Moy_scrs | | durationFixSD | | |
| ET_lat_scrs | | | | |

When validated on the 10 final test subjects, the method had a true positive rate of 0.744, a false positive rate of 0.262, a precision of 0.703, recall of 0.744, an F-measure of 0.744, a Matthews correlation coefficient of 0.485 and areas of 0.826 and 0.845 under, respectively, the receiver operating characteristic and precision recall curves.

In the second example, the data processing device 1 determined the discomfort level index value using a J48 algorithm, based on the parameters and corresponding time windows presented in Table 2:

| Electro-dermal | Cardiac | Eye-tracking | Behavioral | Personality |
|---|---|---|---|---|
| 0-15 s | 0-60 s | 0-60 s | 0-120 s | |
| ET_lat_scrs | SDNN | durationFixTot | SDWA | Openness |
| Amp_moy | | | | Agreeability |
| | | | | Extraversion |
| | | | | Neuroticism |

When validated on the 10 final test subjects, the method had then a true positive rate of 0.698, a false positive rate of 0.296, a precision of 0.704, recall of 0.698, an F-measure of 0.698, a Matthews correlation coefficient of 0.402 and areas of 0.739 and 0.704 under, respectively, the receiver operating characteristic and precision recall curves.

In the third example, the data processing device 1 determined the discomfort level index value using a naive Bayesian algorithm, based on the parameters and corresponding time windows presented in Table 3:

| Electro-dermal | Cardiac | Eye-tracking | Behavioral | Personality |
|---|---|---|---|---|
| 0-15 s | 0-60 s | 0-30 s | 0-60 s | |
| N_scrs | SDNN | durationFixTot | SDLP | Openness |
| | | | SRR | Neuroticism |

When validated on the 10 final test subjects, the method had then a true positive rate of 0.744, a false positive rate of 0.262, a precision of 0.747, recall of 0.744, an F-measure of 0.742, a Matthews correlation coefficient of 0.486 and areas of 0.743 and 0.757 under, respectively, the receiver operating characteristic and precision recall curves.

Those skilled in the art will recognize that the present invention may be manifested in a variety of forms other than the specific embodiments described and contemplated herein. Accordingly, departure in form and detail may be made without departing from the scope of the present invention as defined in the appended claims.

## Claims

1. A data processing device (1) configured to be connected to one or more physiological sensors (2a,2b,2c) for receiving physiological data of a user, to one or more behavioral sensors (3) for receiving behavioral data of the user and to a vehicle context detection unit (4) for receiving vehicle context information, and to determine a discomfort level index value based on a personality profile of the user together with at least the vehicle context information and physiological and behavioral data received within corresponding time windows.

2. The data processing device (1) according to claim 1, configured to be also connected to an emotional state detection unit (8) for receiving emotional state information concerning the user, and to determine the discomfort level index value based also on the emotional state information concerning the user.

3. The data processing device (1) according to claim 2, wherein the emotional state detection unit (8) is configured to detect emotional cues in images and/or sound received from a camera (9a) and/or a microphone (9b) connected thereto.

4. The data processing device (1) according to any one of claims 1 to 3, wherein the personality profile comprises an index value associated to each of one or more personality traits.

5. The data processing device (1) according to claim 4, wherein the one or more personality traits include openness, agreeableness, extraversion, neuroticism, conscientiousness, and/or trait anxiety.

6. The data processing device (1) according to any one of the previous claims, configured to apply a learning classification algorithm for determining the discomfort level index value.

7. The data processing device (1) according to claim 6, wherein the learning classification algorithm is any one of random forest, J48, naïve Bayesian, deep neural network, or recurrent neural network algorithms.

8. The data processing device (1) according to any one of the previous claims, wherein the one or more physiological sensors (2a,2b,2c) include one or more electro-dermal sensors (2a).

9. The data processing device (1) according to claim 8, configured to receive, as physiological data from the one or more electro-dermal sensors (2a), occurrence, amplitude and/or latency of phasic skin conductivity responses.

10. The data processing device (1) according to any one of the previous claims, wherein the one or more physiological sensors (2a,2b,2c) include one or more cardiac sensors (2b).

11. The data processing device (1) according to claim 10, configured to receive RR intervals as physiological data from the one or more cardiac sensors (2b).

12. The data processing device (1) according to any one of the previous claims, wherein the one or more physiological sensors (2a,2b,2c) include one or more eye tracking sensors (2c).

13. The data processing device (1) according to claim 12, configured to receive occurrence and/or duration of eye fixations as physiological data from the one or more eye tracking sensors (2c).

14. The data processing device (1) according to any previous claim, also configured to output a warning and/or trigger user assistance if the discomfort level index value exceeds a predetermined threshold.

15. A human-machine interface system (20) comprising the data processing device (1) according to any previous claim, as well as the one or more physiological sensors (2a,2b,2c), the one or more behavioral sensors (3), and the vehicle context detection unit (4), each connected to the data processing device (1).

16. A vehicle (10) comprising the human-machine interface system (20) according to any one of claims 9 to 15.

17. A computer-implemented method for evaluating user discomfort, comprising the steps of:
receiving physiological data of a user from one or more physiological sensors (2a,2b,2c);
receiving behavioral data of the user from one or more behavioral sensors (3);
receiving vehicle context information from a vehicle context detection unit (4); and
determining a discomfort level index value based on a personality profile of the user, together with at least the physiological and behavioral data and vehicle context information received within corresponding time windows.

18. A computer program comprising instructions which, when the program is executed by a computer, cause the computer to perform the computer-implemented method according to claim 17.

19. A computer-readable medium comprising instructions which, when executed by a computer, cause the computer to carry out the computer-implemented method according to claim 17.

## Patentansprüche

1. Datenverarbeitungsvorrichtung (1), die dazu ausgestaltet ist, mit einem oder mehreren physiologischen Sensoren (2a, 2b, 2c) zum Empfangen von physiologischen Daten eines Benutzers, mit einem oder mehreren Verhaltenssensoren (3) zum Empfangen von Verhaltensdaten des Benutzers und mit einer Fahrzeugkontextdetektionseinheit (4) zum Empfangen von Fahrzeugkontextinformationen verbunden zu sein und einen Unbequemlichkeitspegel-Indexwert auf Grundlage eines Persönlichkeitsprofils des Benutzers zusammen mit mindestens den Fahrzeugkontextinformationen und physiologischen Daten und Verhaltensdaten, die innerhalb entsprechender Zeitfenster empfangen werden, zu bestimmen.

2. Datenverarbeitungsvorrichtung (1) nach Anspruch 1, die dazu ausgestaltet ist, auch mit einer Emotionszustandsdetektionseinheit (8) zum Empfangen von Emotionszustandsinformationen, die den Benutzer betreffen, verbunden zu sein und den Unbequemlichkeitspegel-Indexwert auch auf Grundlage der Emotionszustandsinformationen, die den Benutzer betreffen, zu bestimmen.

3. Datenverarbeitungsvorrichtung (1) nach Anspruch 2, wobei die Emotionszustandsdetektionseinheit (8) dazu ausgestaltet ist, Emotionshinweise in Bildern und/oder Tönen zu detektieren, die von einer Kamera (9a) und/oder einem Mikrofon (9b), die/das damit verbunden ist, empfangen werden.

4. Datenverarbeitungsvorrichtung (1) nach einem der Ansprüche 1 bis 3, wobei das Persönlichkeitsprofil einen Indexwert umfasst, der jeder von einer oder mehreren Persönlichkeitseigenschaften zugeordnet ist.

5. Datenverarbeitungsvorrichtung (1) nach Anspruch 4, wobei die eine oder die mehreren Persönlichkeitseigenschaften Offenheit, Verträglichkeit, Extravertiertheit, Neurotizismus, Gewissenhaftigkeit und/oder Eigenschaftsangst beinhaltet/n.

6. Datenverarbeitungsvorrichtung (1) nach einem der vorhergehenden Ansprüche, die dazu ausgestaltet ist, einen Lernklassifizierungsalgorithmus zum Bestimmen des Unbequemlichkeitspegel-Indexwerts anzuwenden.

7. Datenverarbeitungsvorrichtung (1) nach Anspruch 6, wobei der Lernklassifizierungsalgorithmus ein beliebiger von Random-Forest-Algorithmus, J48-Algorithmus, Naivem-Bayes-Algorithmus, einem Algorithmus eines tiefen neuronalen Netzes oder eines Algorithmus eines rekurrenten neuronalen Netzes ist.

8. Datenverarbeitungsvorrichtung (1) nach einem der vorhergehenden Ansprüche, wobei der eine oder die mehreren physiologischen Sensoren (2a, 2b, 2c) einen oder mehrere elektrodermale Sensoren (2a) beinhaltet/n.

9. Datenverarbeitungsvorrichtung (1) nach Anspruch 8, die dazu ausgestaltet ist, Auftreten, Amplitude und/oder Latenz von phasischen Hautleitfähigkeitsreaktionen als physiologische Daten von dem einen oder den mehreren elektrodermalen Sensoren (2a) zu empfangen.

10. Datenverarbeitungsvorrichtung (1) nach einem der vorhergehenden Ansprüche, wobei der eine oder die mehreren physiologischen Sensoren (2a, 2b, 2c) einen oder mehrere Herzsensoren (2b) beinhaltet/n.

11. Datenverarbeitungsvorrichtung (1) nach Anspruch 10, die dazu ausgestaltet ist, RR-Intervalle als physiologische Daten von dem einen oder den mehreren Herzsensoren (2b) zu empfangen.

12. Datenverarbeitungsvorrichtung (1) nach einem der vorhergehenden Ansprüche, wobei der eine oder die mehreren physiologischen Sensoren (2a, 2b, 2c) einen oder mehrere Augenverfolgungssensoren (2c) beinhaltet/n.

13. Datenverarbeitungsvorrichtung (1) nach Anspruch 12, die dazu ausgestaltet ist, Auftreten und/oder Dauer von Augenfixierungen als physiologische Daten von dem einen oder den mehreren Augenverfolgungssensoren (2c) zu empfangen.

14. Datenverarbeitungsvorrichtung (1) nach einem der vorhergehenden Ansprüche, die zudem dazu ausgestaltet ist, eine Warnung auszugeben und/oder Benutzerunterstützung auszulösen, wenn der Unbequemlichkeitspegel-Indexwert einen vorbestimmten Schwellenwert überschreitet.

15. Mensch-Maschine-Schnittstellensystem (20), umfassend die Datenverarbeitungsvorrichtung (1) nach einem der vorhergehenden Ansprüche, sowie den einen oder die mehreren physiologischen Sensoren (2a, 2b, 2c), den einen oder die mehreren Verhaltenssensoren (3) und die Fahrzeugkontextdetektionseinheit (4), die jeweils mit der Datenverarbeitungsvorrichtung (1) verbunden sind.

16. Fahrzeug (10), umfassend das Mensch-Maschine-Schnittstellensystem (20) nach einem der Ansprüche 9 bis 15.

17. Computerimplementiertes Verfahren zum Bewerten von Benutzerkomfort, umfassend die Schritte des:
Empfangens von physiologischen Daten eines Benutzers von einem oder mehreren physiologischen Sensoren (2a, 2b, 2c),
Empfangens von Verhaltensdaten des Benutzers von einem oder mehreren Verhaltenssensoren (3),
Empfangens von Fahrzeugkontextinformationen von einer Fahrzeugkontextdetektionseinheit (4), und
Bestimmens eines Unbequemlichkeitspegel-Indexwerts auf Grundlage eines Persönlichkeitsprofils des Benutzers zusammen mit mindestens den physiologischen Daten und Verhaltensdaten und Fahrzeugkontextinformationen, die innerhalb entsprechender Zeitfenster empfangen werden.

18. Computerprogramm, umfassend Anweisungen, die, wenn das Programm durch einen Computer ausgeführt wird, den Computer veranlassen, das computerimplementierte Verfahren nach Anspruch 17 durchzuführen.

19. Computerlesbares Medium, umfassend Anweisungen, die, wenn sie durch einen Computer ausgeführt werden, den Computer veranlassen, das computerimplementierte Verfahren nach Anspruch 17 auszuführen.

## Revendications

1. Dispositif de traitement de données (1) configuré pour être relié à un ou plusieurs capteurs physiologiques (2a, 2b, 2c) pour recevoir des données physiologiques d'un utilisateur, à un ou plusieurs capteurs comportementaux (3) pour recevoir des données comportementales de l'utilisateur et à une unité de détection de contexte de véhicule (4) pour recevoir de l'information de contexte de véhicule, et pour déterminer une valeur d'indice de niveau d'inconfort sur la base d'un profil de personnalité de l'utilisateur avec au moins l'information de contexte de véhicule et les données physiologiques et comportementales reçues dans des fenêtres de temps correspondantes.

2. Dispositif de traitement de données (1) selon la revendication 1, configuré pour être également relié à une unité de détection d'état émotionnel (8) pour recevoir de l'information d'état émotionnel concernant l'utilisateur, et pour déterminer la valeur d'indice de niveau d'inconfort sur la base également de l'information d'état émotionnel concernant l'utilisateur.

3. Dispositif de traitement de données (1) selon la revendication 2, dans lequel l'unité de détection d'état émotionnel (8) est configurée pour détecter des signaux émotionnels dans les images et/ou du son reçus d'une caméra (9a) et/ou d'un microphone (9b) reliés à celle-ci.

4. Dispositif de traitement de données (1) selon l'une quelconque des revendications 1 à 3, dans lequel le profil de personnalité comprend une valeur d'indice associée à chacun d'un ou plusieurs traits de personnalité.

5. Dispositif de traitement de données (1) selon la revendication 4, dans lequel les un ou plusieurs traits de personnalité comprennent l'ouverture, l'agréabilité, l'extraversion, le neuroticisme, la conscienciosité, et/ou l'anxiété chronique.

6. Dispositif de traitement de données (1) selon l'une quelconque des revendications précédentes, configuré pour appliquer un algorithme de classification d'apprentissage pour déterminer la valeur d'indice de niveau d'inconfort.

7. Dispositif de traitement de données (1) selon la revendication 6, dans lequel l'algorithme de classification d'apprentissage est l'un quelconque des algorithmes de forêt aléatoire, J48, Bayésien naïf, de réseau neuronal profond ou de réseau neuronal récurrent.

8. Dispositif de traitement de données (1) selon l'une quelconque des revendications précédentes, dans lequel les un ou plusieurs capteurs physiologiques (2a, 2b, 2c) comprennent un ou plusieurs capteurs électro-cutanés (2a) .

9. Dispositif de traitement de données (1) selon la revendication 8, configuré pour recevoir, en tant que données physiologiques provenant des un ou plusieurs capteurs électro-cutanés (2a), une occurrence, une amplitude et/ou une latence de réponses de conductivité de peau phasique.

10. Dispositif de traitement de données (1) selon l'une quelconque des revendications précédentes, dans lequel les un ou plusieurs capteurs physiologiques (2a, 2b, 2c) comprennent un ou plusieurs capteurs cardiaques (2b).

11. Dispositif de traitement de données (1) selon la revendication 10, configuré pour recevoir des intervalles RR en tant que données physiologiques provenant des un ou plusieurs capteurs cardiaques (2b).

12. Dispositif de traitement de données (1) selon l'une quelconque des revendications précédentes, dans lequel les un ou plusieurs capteurs physiologiques (2a, 2b, 2c) comprennent un ou plusieurs capteurs de suivi d'œil (2c) .

13. Dispositif de traitement de données (1) selon la revendication 12, configuré pour recevoir une occurrence et/ou une durée de fixations d'œil en tant que données physiologiques provenant des un ou plusieurs capteurs de suivi d'œil (2c).

14. Dispositif de traitement de données (1) selon l'une quelconque des revendications précédentes, également configuré pour délivrer une alarme et/ou déclencher une assistance à l'utilisateur si la valeur d'indice de niveau d'inconfort dépasse un seuil prédéterminé.

15. Système d'interface homme-machine (20) comprenant le dispositif de traitement de données (1) selon l'une quelconque des revendications précédentes, ainsi que les un ou plusieurs capteurs physiologiques (2a, 2b, 2c), les un ou plusieurs capteurs comportementaux (3), et l'unité de détection de contexte de véhicule (4), chacun relié au dispositif de traitement de données (1).

16. Véhicule (10) comprenant le système d'interface homme-machine (20) selon l'une quelconque des revendications 9 à 15.

17. Procédé mis en œuvre par ordinateur pour évaluer un inconfort d'utilisateur, comprenant les étapes de :
réception de données physiologiques d'un utilisateur provenant d'un ou plusieurs capteurs physiologiques (2a, 2b, 2c) ;
réception de données comportementales de l'utilisateur provenant d'un ou plusieurs capteurs comportementaux (3) ;
réception d'information de contexte de véhicule provenant d'une unité de détection de contexte de véhicule (4) ; et
détermination d'une valeur d'indice de niveau d'inconfort sur la base d'un profil de personnalité de l'utilisateur, avec au moins les données physiologiques et comportementales et l'information de contexte de véhicule reçues dans les fenêtres de temps correspondantes.

18. Programme informatique comprenant des instructions qui, quand le programme est exécuté par un ordinateur, amènent l'ordinateur à exécuter le procédé mis en œuvre par ordinateur selon la revendication 17.

19. Support lisible par ordinateur comprenant des instructions qui, lorsqu'elles sont exécutées par un ordinateur, amènent l'ordinateur à exécuter le procédé mis en œuvre par ordinateur selon la revendication 17.
